# EUROPEAN PATENT SPECIFICATION

(11) **EP 4 233 949 B1**
(45) Date of publication and mention of the grant of the patent: **23.04.2025**
(21) Application number: 22158382.6
(22) Date of filing: 24.02.2022
(51) Int. Cl.: A61M 11/06, A61M 16/20, A61M 16/10, A61M 16/12, A61M 16/08, A61M 16/06, A61M 16/00

(54) **VENTURI DEVICE FOR REGULATING THE OXYGEN FRACTION IN RESPIRATORY THERAPIES**
VENTURI-VORRICHTUNG ZUM REGULIEREN DES SAUERSTOFFANTEILS IN ATEMTHERAPIEN
DISPOSITIF VENTURI POUR RÉGULER LA FRACTION D'OXYGÈNE DANS DES THÉRAPIES RESPIRATOIRES

(43) Date of publication of application: 30.08.2023
(73) Proprietor: Air Liquide Medical Systems, 92182 Antony Cedex (FR); Air Liquide Medical Systems S.r.l., 20152 Milano (IT)
(72) Inventor: ZADRA, Davide, 25073 Bovezzo (IT); ALBERICI, Luca, 25073 Bovezzo (IT); SANDONI, Giuseppe, 25073 Bovezzo (IT); LESIMPLE, Arnaud, 92182 Antony Cedex (FR); DE BEAUFORT, Eloise, 92182 Antony Cedex (FR); BROC, Alexandre, 92182 Antony Cedex (FR); PROUVEZ, Nathan, 92182 Antony Cedex (FR)
(74) Representative: Air Liquide

(56) References cited:
- WO-A1-2021/216262
- GB-A- 805 941
- US-A- 3 913 607
- US-B2- 9 498 592

## Description

The present disclosure concerns a venturi device useable for regulating the fraction of oxygen required for treating a patient, i.e. person, suffering from respiratory troubles, such as a person infected by a coronavirus, for example the Covid-19 virus or one of its variants, involving pulmonary troubles, failures or the like, leading to hypoxemia or the like.

People can suffer from respiratory troubles due to a failure of their lungs that can be the consequence of for instance an infection by microorganisms, typically bacteria and/or viruses, like a coronavirus, such as the Covid-19 virus including its variants.

When the pulmonary failures of infected persons lead to a lack of oxygen in their blood, i.e. a hypoxemia or the like, they have to be hospitalized for treating their pulmonary failures in providing them an oxygen-containing respiratory gas, such as an air/oxygen mixture or pure oxygen.

With severely ill patients, respiratory assistance devices, also called medical ventilators, respirators or the like, are used for providing the oxygen-containing gas.

However, such medical ventilators are not suitable or not well-adapted to less affected patients, i.e. "light" cases, which represents the majority of the patients.

Further, many medical ventilators are not usable either in the field, for instance in emergency units, such as SAMU, SDIS or the like, as they are too cumbersome and not easy to handle and carry, especially when they have to be coupled to an oxygen source, namely a gas cylinder.

These are the reasons why, simpler non-invasive ventilation devices have been proposed, such as pneumatically-actuated devices comprising simple components fluidly cooperating together, such as a hollow body including gas inlet(s) and outlet(s), gas reservoir, flexible hose, respiratory interface... Despite their simple architecture, such non-invasive ventilation devices work rather well for providing oxygen or air/oxygen mixtures to numerous patients.

For regulating the quantity or fraction of oxygen delivered to the patients, which depends on their needs, it is necessary to regulate the flow of oxygen provided by the oxygen source, such as an O₂ gas cylinder or a wall O₂-connector/plug, and its subsequent mixing with air, if required, for delivering pure oxygen or an air/O₂ mixture.

This may be done by a venturi device that is plugged between the oxygen source and a non-invasive ventilation device, and further fed with ambient air, such as disclosed by EP-A-1852137.

As the amount or fraction of oxygen provided to the patient may vary in the course of the treatment, some venturi devices comprise a regulation system for selecting the gas that should be delivered, namely pure oxygen or an air/oxygen mixture.

Known regulation systems for venturi devices are actuatable by the user between :
- an open position wherein oxygen can be mixed with air for obtaining and delivering an oxygen/air mixture (i.e. O₂ concentration less than 100% vol.), and
- a closed position wherein any mixing of air and O₂ is prohibited thereby delivering only pure oxygen (i.e. O₂ concentration of 100% vol.).

For doing so, a regulation system generally comprises two mobile parts that are axially-slidable, i.e. mobile in axial translation, one toward (or away from) the other so that the user can easily select the desired position, namely an open or a closed position, by axially-sliding one of those mobile parts, thereby allowing or prohibiting any mixing of oxygen with air.

However, such a regulation system is not ideal as it can lead to an accidental sliding of the mobile parts involving a wrong position setting and hence a delivery of a gas that does not have the desired composition, for instance oxygen in lieu of an air/O₂ mixture, or vice versa. Moreover, most existing systems are not able to provide pure oxygen (i.e. O₂ concentration of 100% vol.) in a closed position as leaks often occur thereby allowing air to enter and to lower the O₂ concentration delivered to the patient.

One can easily understand that delivering a wrong gas composition can be a serious problem for a patient, when said patient needs to inhale a specific amount of oxygen for treating a given respiratory trouble or condition affecting said patient. For example, delivering an air/oxygen mixture in lieu of pure oxygen to a patient may lead to a severe hypoxemia and, in extreme cases, to the death of the patient. Conversely, delivering pure oxygen in lieu of an air/oxygen mixture may lead to hyperoxia that may be detrimental to the patient.

Other examples of fluid mixing devices including venturi elements useable for instance for prodiving oxygen mixtures to patients are given by WO-A-2021/216262, GB-A-805,941, US-A-9,498,592 and US-A-3,913,607.

The invention is defined in the appended claims. A goal of the present disclosure is to provide an improved venturi device that overcomes said problem to better control the fraction of oxygen provided to a patient.

The solution according to the invention relates to a venturi device for delivering oxygen or an air/oxygen mixture (i.e. oxygen-enriched air) comprising :
- a main body comprising an inner compartment (i.e. inner hollow volume) and a venturi nozzle arranged in said inner compartment, and
- a rear member comprising an oxygen inlet, i.e. an oxygen port, for providing oxygen and at least one air inlet, i.e. an air port, for providing air, and a mixing chamber for mixing therein oxygen and air provided by said oxygen inlet and said at least one air inlet,

wherein the rear member is mobile with respect to the main body between at least :
   o a closed position wherein the rear member provides oxygen to the venturi nozzle, and
   o an open position wherein the rear member provides an air/oxygen mixture to the venturi nozzle, said air/oxygen mixture being obtained in the mixing chamber of the rear member,
characterized in that the rear member is mobile with respect to the main body according to a helicoidal motion for allowing a user to manually select a desired open or closed position.

Depending on the embodiment, the venturi device according to the present disclosure can comprise one or several of the following features:
- The rear member is a gas-providing member as it provides gas, namely oxygen and/or air.
- The closed position and the open position are stable positions.
- The rear member cooperates with the main body.
- The rear member (or the main body) can move back and forth with respect to the main body (or the rear member), while actuated according to a helicoidal motion by the user, i.e. while turned clockwise or counterclockwise by the user.
- The guiding members and the elongated grooves are configured so that each guiding member travels into one corresponding elongated groove, when the rear member is actuated (i.e. turned), clockwise or counterclockwise, with respect to the main body, or vice versa, by a user.
- The rear member cooperates with the venturi nozzle of the main body for providing oxygen or an air-oxygen mixture to the venturi nozzle, in particular to the nozzle inlet of the venturi nozzle.
- The rear member is mobile with respect to the main body according to a helicoidal motion, i.e. along a helicoidal path, namely a combination of a rotation and a translation, when actuated by a user, i.e. it is turned or rotated clockwise or anti-clockwise.
- The main body comprises at least one elongated groove and the rear member comprises at least one guiding member, or vice versa.
- Said at least one guiding member is inserted into said at least one elongated groove so as to cooperate with said at least one elongated groove for guiding the motion, in particular a helicoidal motion, of the rear member with respect to the main body, while the rear member is actuated, i.e. at least rotated / turned around axis (XX), by a user with respect to the main body.
- Preferably, the main body comprises at least two elongated grooves and the rear member comprises at least two guiding members, or vice versa, in particular two elongated grooves and two guiding members, each guiding member cooperating with one elongated groove.
- The two elongated grooves are diametrically-oppositely arranged on the main body.
- The two guiding members are diametrically-oppositely arranged on the rear member.
- Each elongated groove constitutes a guide or path that guides the guiding member, while the venturi device is actuated by the user.
- Each elongated groove is arranged to form a slot traversing the peripheral wall of the main body, i.e. a traversing slot or the like, preferably traversing the peripheral wall of the inlet chamber of the main body.
- Each guiding member comprises or is a protrusion arranged on the peripheral outer surface of a front portion of the rear member.
- Each protrusion forms a dent, a pin or any other similar small structure projecting away from the peripheral outer surface of the front portion of the rear member.
- The venturi nozzle comprises a nozzle inlet and a nozzle outlet.
- The venturi nozzle further comprises an inner nozzle channel for conveying the gas, i.e. oxygen or air/oxygen, from the nozzle inlet to the nozzle outlet.
- The inner nozzle channel axially-traverses the venturi nozzle.
- The inner compartment is divided by an inner wall in 2 sub-compartments, namely an inlet chamber, also called upstream chamber, and an outlet chamber, also called downstream chamber.
- The main body has an axially-elongated shape, i.e. along axis (XX).
- The venturi nozzle is arranged through, i.e. traverses, said inner wall.
- The inner wall arranged in the inner compartment of the main body has a ring shape or the like.
- The inner wall is radially-arranged in the inner compartment of the main body, i.e. the inner wall projects radially in the inner compartment.
- The outlet chamber comprises the gas outlet of the venturi nozzle.
- The outlet chamber is open to, i.e. it fluidly communicates with, the exterior of the main body.
- The inlet chamber is open to, i.e. it fluidly communicates with, the mixing chamber of the rear member.
- The inlet chamber of the inner compartment of the main body is in fluid communication with the mixing chamber of the rear member.
- The oxygen-containing gas (i.e. O₂ or air/O₂) can only travel through the venturi nozzle for reaching the gas outlet. In other words, there is no direct fluid communication between the inlet chamber and the outlet chamber, except via the venturi nozzle.
- The main body has a generally-tubular shape, preferably a cylindrical shape.
- The main body has an outer diameter of about between 25 and 40 mm, and a length of about between 60 and 110 mm.
- A front portion of the rear member is inserted into at least a part of the inlet chamber of the main body.
- The rear member is at least partially inserted into the main body so as to be mobile according to a helicoidal motion therein, while actuated by a user.
- The front portion of the rear member is cylindrical, i.e. tubular, and at least a part of the inlet chamber of the main body is cylindrical, so that said cylindrical front portion of the rear member is mobile according to a helicoidal motion n into said cylindrical part of the inlet chamber of the main body.
- The rear member comprises a tubular portion closed at one end by a blind bottom, i.e. a tubular portion terminated by a closed end.
- The rear member comprises a cup-like shape portion comprising the tubular portion terminated by the closed end.
- The rear member comprises a cylindrical body terminated by an open end at a first end and a closed end, i.e. a blind bottom, at a second end.
- The rear member has a generally-tubular shape having an outer diameter of about between 20 and 40 mm, and a length of about between 40 and 70 mm.
- The rear member comprises, i.e. is traversed by, an oxygen feeding conduct comprising an outer conduct portion (i.e. upstream portion) and an inner conduct portion (i.e. downstream portion), for providing oxygen.
- The oxygen feeding conduct is axially-arranged in the rear member.
- The closed end, i.e. the blind bottom or closing wall, of the rear member is traversed by the oxygen feeding conduct.
- The outer conduct portion of the oxygen feeding conduct comprises the oxygen inlet.
- The inner conduct portion of the oxygen feeding conduct comprises an oxygen delivery port.
- The oxygen feeding conduct is axially-traversed by a lumen for conveying oxygen from the oxygen inlet to the oxygen delivery port thereby providing oxygen coming from an external oxygen source.
- The outer conduct portion of the oxygen feeding conduct comprises connector means, such as a specific outer shape, for fluidly connecting an oxygen line thereto, preferably an oxygen line fluidly connected to an external oxygen source, such as an oxygen gas cylinder or an oxygen wall-plug fed by a gas network.
- The inner conduct portion of the oxygen feeding conduct is arranged so as to (axially-) project into the mixing chamber of the rear member.
- The oxygen delivery port is arranged at a proximal free end of the inner conduct portion of the oxygen feeding conduct.
- The outer conduct portion of the oxygen feeding conduct is arranged so as to (axially-) project outwardly of the rear member, i.e. outside of it.
- The oxygen inlet is arranged at a distal free end of the outer conduct portion of the oxygen feeding conduct.
- The oxygen feeding conduct is in fluid communication, in particular the lumen of the inner and outer conduct portions, with the mixing chamber, when the rear member is in the open position.
- The oxygen feeding conduct is in fluid communication, in particular the lumen of the inner and outer conduct portions, with the inner nozzle channel of the venturi nozzle, when the rear member is in the closed position.
- The mixing chamber of the rear member has an inner cylindrical shape.
- The outer diameter of the cylindrical front portion of the rear member is approximately equal to the inner diameter of the cylindrical part of the inlet chamber of the main body, i.e. the outer diameter of the cylindrical front portion is slightly less than the inner diameter of the cylindrical part of the inlet chamber so that the rear member is mobile according to a helicoidal motion, into said main body.
- The rear member comprises several air inlets or air ports for providing air.
- Several air inlets are arranged around the oxygen feeding conduct, in particular in the blind bottom.
- The inlet chamber of the main body is delimited by a peripheral wall.
- The inlet chamber of the main body is in fluid communication with the mixing chamber of the rear element.
- The venturi nozzle comprises a first portion comprising the nozzle inlet.
- The first portion is a cylindrical or a convergent portion.
- The venturi nozzle further comprises a second portion comprising the nozzle outlet.
- The second portion is a divergent portion, i.e. it has a divergent inner shape (i.e. an increasing inner diameter).
- The first portion is fluidly connected to the second portion of the venturi nozzle.
- The divergent portion is arranged between the first portion of the nozzle channel (i.e. cylindrical portion) and the nozzle outlet, i.e. a gas exit of the venturi nozzle.
- The first portion and the second portion of the venturi nozzle form the inner nozzle channel for conveying the gas, i.e. oxygen or air/oxygen.
- The nozzle inlet is located at the entry of the inner nozzle channel and the nozzle outlet is located at the exit of the inner nozzle channel so that the nozzle inlet is fluidly connected to the nozzle outlet via the inner nozzle channel.
- The gas circulates into the venturi nozzle from the first portion, i.e. a gas entry portion, having preferably a circular or convergent shape, to the second portion, i.e. a gas exit portion, having preferably a divergent shape.
- In the closed position, the proximal free end of the inner conduct portion of the oxygen feeding conduct of the rear member is inserted into the first portion of the inner nozzle channel of the venturi nozzle so as to deliver oxygen directly into the venturi nozzle.
- In the open position, the proximal free end of the inner conduct portion of the oxygen feeding conduct of the rear member is not inserted into the first portion of the inner nozzle channel of the venturi nozzle, but delivers a flow of oxygen into the mixing chamber so as to generate an air/oxygen mixture into the mixing chamber of the venturi nozzle.
- In the open position, air provided by the air inlet(s) is sucked into the mixing chamber of the rear member by the flow of oxygen provided by the oxygen feeding conduct of the rear member.
- Each elongated groove of the main body, preferably each elongated slot, comprises a curved linear portion terminated by two end lodgings.
- Each end lodging comprises an abutment, preferably each abutment is located at a site of junction between the elongated groove and each end lodging.
- The curved linear portion of each elongated groove, preferably of each elongated slot, is diagonally-arranged in the peripheral wall of the main body, namely in the peripheral wall of the inlet chamber of the main body.
- Each elongated groove, preferably of each elongated slot, has a length of about between 20 mm and 30 mm.
- Each elongated groove, preferably of each elongated slot, has a width of about between 3.5 mm and 5 mm.
- Each guiding member, i.e. each a protrusion, has a height of about between 1.5 and 3 mm.

The present disclosure also concerns an installation for providing oxygen or an air/oxygen mixture to a patient comprising a gas reservoir, a flexible hose, a respiratory interface and a connecting hollow body, the gas reservoir and the flexible hose being fluidly connected to the connecting hollow body, and the respiratory interface being fluidly connected to the flexible hose, characterized in that a venturi device according to the present disclosure is further fluidly connected to the connecting hollow body for delivering oxygen or the air/oxygen mixture to said connecting hollow body.

Depending on the embodiment, the installation according to the present disclosure can comprise one or several of the following features:
- an oxygen line is fluidly connected to the venturi device for providing oxygen thereto.
- the oxygen line comprises a gas conduit, preferably a gas conduit equipped with one or several connectors or the like, for fluidly connecting the oxygen line to the venturi device and/or to an oxygen source, such as an oxygen cylinder or a wall plug delivering oxygen.
- the respiratory interface is a respiratory mask or the like.
- the gas reservoir is a flexible buffer having an inner volume of between about 1 and 50 L (L in water equivalent), typically of at least 15 L, preferably of between 25 and 40 L.

Such an installation according to the present disclosure can be used for providing oxygen or an air/oxygen mixture to a patient, i.e. person, in need thereof, i.e. a patient suffering from respiratory troubles ou disease, such as a person infected by a coronavirus, for example the Covid-19 virus or one of its variants, involving pulmonary troubles, failures or the like, leading to hypoxemia or the like, wherein the fraction of oxygen used for treating said patient is regulated by the venturi device according to the present disclosure.

Embodiments of a venturi device according to the present disclosure are shown in the enclosed illustrative, but not limitative, Figures, among which:
- Figure 1 represents a general view of an embodiment of a venturi device according to the present disclosure,
- Figure 2 is a schematic lateral view of a venturi device according to the present disclosure,
- Figure 3 shows a venturi device according to the present disclosure in the open (on the right side) and closed (on the left side) positions,
- Figure 4 represents the passage of the venturi device of Figure 3 from the closed position to the open position, when actuated by a user, or vice versa,
- Figure 5 shows several lateral views of the venturi device of Figures 3 and 4,
- Figure 6A and 6B represent lateral and sectional views of the venturi device of the present disclosure in the open (Fig. 6A) and closed positions (Fig. 6B), and
- Figure 7 is an enlarged sectional view of the venturi device of the present disclosure in the open corresponding to Fig. 6A.
- Figure 8 shows an installation for providing oxygen or an air/oxygen mixture to a patient including a venturi device according to the present disclosure.

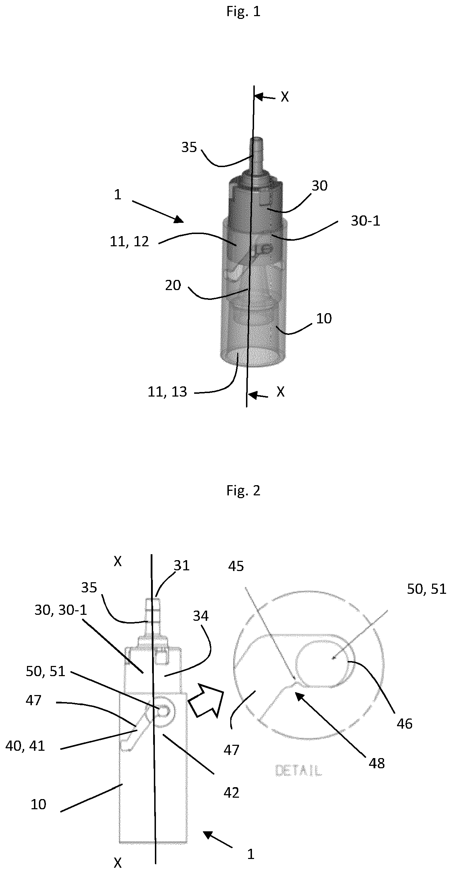

Figure 1 is a general view of an embodiment of a venturi device 1 according to the present disclosure showing in transparency the two main parts or sub-units cooperating together, namely a main body 10 and a rear member 30, i.e. a gas-providing member or part, forming the venturi device 1 according to the present invention, which are mobile with respect to the other for controlling, i.e. selecting, the type of gas that has to be provided by the venturi device 1, i.e. oxygen or an air/oxygen mixture, as below explained.

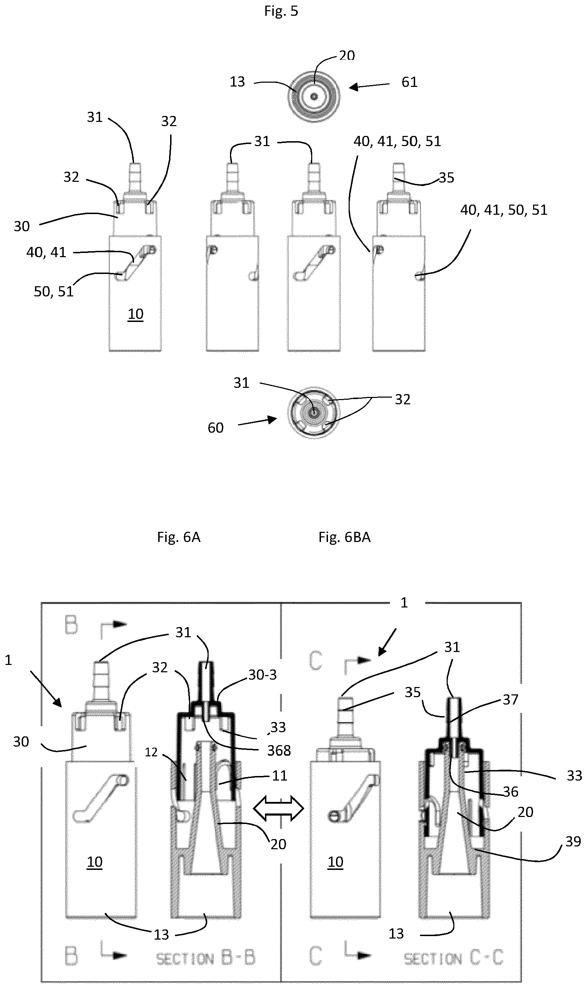

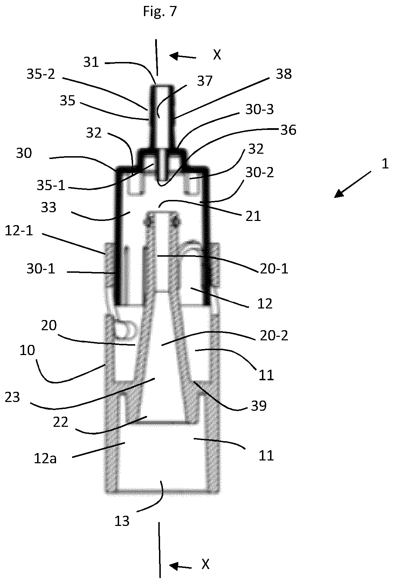

As shown in Fig. 6A&B and 7, the main body 10 comprises an inner compartment 11 comprising a venturi nozzle 20 co-axially arranged in said inner compartment 11. The inner compartment 11 is divided in 2 sub-compartments by an inner wall 39, namely an inlet chamber 12 or upstream chamber, and an outlet chamber 13 or downstream chamber. As the inner compartment 11 has a circular section, i.e. a cylindrical inner-shape, the inner wall 39 has a ring-shape. The outlet chamber 13 is also shown in the bottom view 61 of Fig. 5.

The venturi nozzle 20 is arranged through the inner wall 39 that divides the inner compartment 11. It comprises a nozzle inlet 21 located on the side of the inlet chamber 12 and a nozzle outlet 22 located on the side of the outlet chamber 13, preferably in said outlet chamber 13 as shown in Fig. 6A&B and 7. An inner nozzle channel 20-1, 20-2 is arranged between the nozzle inlet 21 and the nozzle outlet 22 for conveying the gas from the nozzle inlet 21 to the nozzle outlet 22.

More precisely, the venturi nozzle 20 comprises two successive channel portions forming the inner nozzle channel 20-1, 20-2 comprising a first channel portion 20-1 comprising the nozzle inlet 21 and having an inner cylindrical shape, and a second channel portion comprising the nozzle outlet 22 and having an inner divergent shape. The first channel portion 20-1 fluidly connects the nozzle inlet 21 to the second channel portion 20-2. According to another embodiment (not shown), the first channel portion 20-1 can have an inner convergent shape.

The second channel portion 20-2 is arranged between the first channel portion 20-1 and the nozzle outlet 22. As it comprises an inner divergent shape or profile, its inner diameter gradually increases toward the nozzle outlet 22, as shown in Fig. 6A&B and 7.

The gas (i.e. O₂ or air/O₂) enters into the inner nozzle channel 20-1, 20-2 of the venturi nozzle 20 by the nozzle inlet 21, travels successively in the first 20-1 and then second 20-2 channel portions of the inner nozzle channel 20-1, 20-2, and flows out, i.e. leaves, of the venturi nozzle 20 by the nozzle outlet 22.

The main body 10 has an axially-elongated shape, i.e. along axis (XX) shown in Fig. 7, in particular a tubular outer shape, e.g. cylindrical outer shape, but other outer shapes are possible. For instance, the outer diameter of the main body 10 is of about between 25 and 40 mm, and its length is of about between 60 and 110 mm.

Further, the inlet chamber 12 of the main body 10 is in fluid communication with the mixing chamber 33 of the rear member 30 that constitutes the second main part or sub-unit of the venturi device 1 according to the present disclosure.

In the embodiment shown in Fig. 6A&B and 7, the rear member 30 has a generally cup-like shape, but other shapes are possible. It comprises an oxygen inlet 31 for providing oxygen and one or preferably several air inlets 32 for providing air, and the mixing chamber 33 for mixing therein air with the oxygen and provided by said oxygen inlet 31. The oxygen inlet 31 and the air inlets 32, e.g. here four air inlets 32, arranged around the oxygen inlet 31 are better visible in the top view 60 of Fig. 5.

The rear member 30 is mobile with respect to the main body 10, upon actuation of a user, between (at least) a closed position (as shown in Fig. 6B), wherein the rear member 30 cooperates directly with the venturi nozzle 20 for providing oxygen to the venturi nozzle 20, in particular to the inner nozzle channel 20-1, 20-2 of the venturi nozzle 20, and an open position (as shown in Fig. 6A), wherein the rear member 30 cooperates with the venturi nozzle 20 for providing the air/oxygen mixture obtained in the mixing chamber 33 to the inner nozzle channel 20-1, 20-2 of the venturi nozzle 20.

In other words, in the closed position (Fig. 6B), the inner nozzle channel 20-1, 20-2 of the venturi nozzle 20 is fed with oxygen only, i.e. "pure" oxygen, whereas in the closed position (Fig. 6A), the inner nozzle channel 20-1, 20-2 of the venturi nozzle 20 is fed with a mixture of air and oxygen, i.e. an air/oxygen mixture, coming from the mixing chamber 33 of the rear member 30.

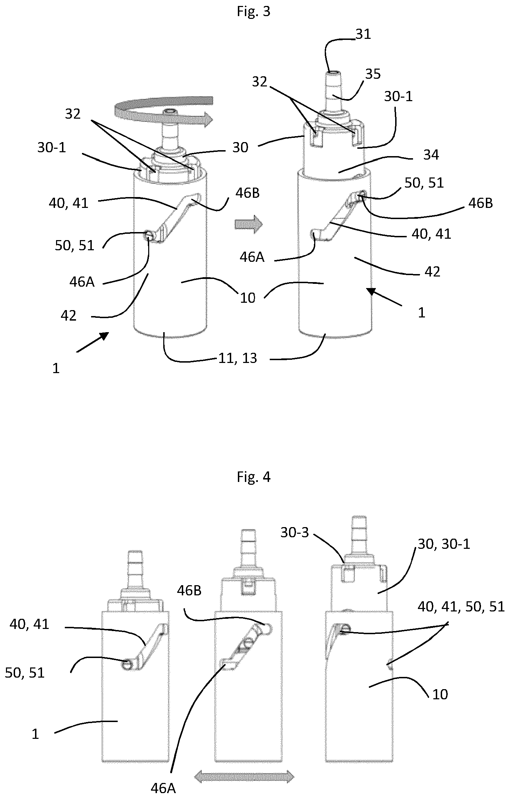

According to the present disclosure, in the goal of avoiding or limiting accidental motions of those mobile parts, i.e. rear member 30 and main body 10, leading to wrong position settings and to a wrong-gas delivery, for instance oxygen in lieu of an air/O₂ mixture, or vice versa, the rear member 30 is not mobile in translation, but rather according to a helicoidal motion with respect to the main body 10, as shown in Figures 3 & 4, for allowing the user to manually select and set a desired open or closed position.

In the context of the present disclosure, "according to a helicoidal motion" means that the selection or setting of the closed or open position is made by the user when turning, clockwise or counterclockwise (cf. Fig. 3 & 4), the rear member 30 with respect to the main body 10 of the venturi device 1 of the invention, or vice versa, thereby obtaining an helicoidal motion of those parts 10, 30, one with respect to the other. In other words, it screws in and out.

In other words, the rear member 30 is helicoidally mobile with respect to the main body 10 when manually-turned clockwise or counterclockwise by a user.

To achieve such a clockwise or counterclockwise motion of those parts, specific structures are provided on the rear member 30 and the main body 10 of the venturi device 1 of the present disclosure.

More precisely, the main body 10 comprises one or several elongated grooves 40 and the rear member 30 comprises one or several guiding members 50, or vice versa, preferably two elongated grooves 40 cooperating with two guiding members 50, as shown in Fig. 1-6A&B, each guiding member 50 being inserted into one of said elongated grooves 40, thereby cooperating with said elongated grooves 40 for guiding the helicoidal motion of the rear member 30 with respect to the main body 10, while the rear member 30 is actuated by a user, i.e. manually rotated/turned around axis (XX), with respect to the main body (10), or vice versa.

As shown in Fig. 4 & 5, the two elongated grooves 40 are arranged in the peripheral wall 42 of the main body 10 and are diametrically-opposed, whereas the two guiding members 50 are diametrically-oppositely arranged on the peripheral surface of the rear member 30.

The elongated grooves 40 constitute guides or paths that guide the guiding members 50, when the venturi device 1 is manually-actuated by the user, i.e. turned clockwise or counterclockwise as shown in Fig. 3 & 4.

As shown in Fig. 4 & 5, the elongated grooves 40 are preferably elongated slots 41 traversing the peripheral wall 42 of the main body 10, in particular the inlet chamber 12 of the main body 10, i.e. traversing slots 41 or the like.

Further, the guiding members 50 comprise or are protrusions 51 arranged on the peripheral outer surface 34 of the rear member 30, preferably the peripheral outer surface 34 of the front portion 30-1 of the rear member 30. Each protrusion 51 forms a dent, a pin or any other similar small structure projecting away from the peripheral outer surface 34 of the rear member 30.

As shown in Fig. 3-5, the elongated grooves 40 of the main body 10, in particular the slots 41, comprise a curved linear portion 47, i.e. a linear segment, arranged in the cylindrical peripheral wall 42 of the main body 10, which is terminated by two end lodgings 46A, 46B that correspond to the open (46B) and closed (46A) positions of the venturi device 1.

The end lodgings 46A, 46B are sized and configured for receiving or lodging the guiding members 50, in particular the protrusions 51. In other words, the protrusions 51 cooperate with the end lodgings 46A, 46B for setting the venturi device 1 in the open (46B) or closed (46A) positions as illustrated in Figure 2.

The curved linear portion 47 of each elongated groove 40, preferably of each elongated slot 41, is diagonally-arranged in the peripheral wall 42 of the main body 10, namely in the peripheral wall of the inlet chamber 12 of the main body 10 as shown in Fig. 3-5.

For ensuring a stable maintaining of the venturi device 1 in said open or closed position, it is provided, in each end lodging 46A, 46B, an abutment 45 as visible in Figure 2, that is located at a site of junction 48 between of the curved linear portion 47 of the elongated grooves/slots 40, 41 and each end lodgings 46A, 46B.

As shown in Figure 2, said abutments 45 can be little wall structures or similar projecting in each end lodging 46A, 46B. The role of those abutments 45 is to block or to retain the protrusions 51 in the end lodgings 46A, 46B for setting the venturi device 1 in the open (46B) or in the closed (46A) positions as illustrated in Figures 2-5. In other words, those abutments 45 are safety blocks or the like.

For instance, when the venturi device 1 in the closed position with the two protrusions 51 lodged and blocked in the first end lodgings 46A corresponding to said closed position, as illustrated in Fig. 3 (left side), and has to be set in the open position, the user actuates, namely rotates counterclockwise, for instance the rear member 30 so that the two protrusions 51 are helicoidally guided by the two slots 41, in particular the linear curved portions of the slots 41, until they reach the second end lodgings 46B corresponding to the open position, as illustrated in Fig. 3 (right side).

Once lodged in the second end lodgings 46B, the two protrusions 51 are blocked and retained therein by the abutments 45, as better shown in Fig. 2, that shows an enlarged view of a second end lodging 46B with a protrusion 51 retained therein by an abutment 45. The abutments 45 prohibit that the protrusions 51 accidentally get out of the end lodgings 46A, 46B thereby ensuring stable open or closed positions of the venturi device 1.

For instance, the elongated grooves 40, e.g. the elongated slots 41, each have a length of about between 20 and 30 mm, and/or a width of about between 3.5 and 5 mm. Further, the guiding members 50, e.g. the protrusions 51, have a height of about between 1.5 and 3 mm.

Furthermore, as visible in Fig. 6A&6B and 7, for allowing the rear member 30 to rotate with respect to the main body 10 of the venturi device 1 of the present disclosure, a front portion 30-1 of the rear member 30 is inserted into at least a part of the inlet chamber 12 of the main body 10.

Further, said front portion 30-1 of the rear member 30 is externally cylindrical, i.e. tubular, and at least a part 12-1 of the inlet chamber 12 of the main body 10 is internally cylindrical, so that said cylindrical front portion 30-1 of the rear member 30 can be rotate in said cylindrical part 12-1 of the inlet chamber 12 of the main body 10 as shown in Fig. 6A&6B and 7. Preferably, the outer diameter Do of said front portion 30-1 of the rear member 30 is less but approximately equal to the inner diameter Di of the cylindrical part 12-1 of the inlet chamber 12 of the main body 10 so that the helicoidal motion is allowed, i.e. Do<Di.

Generally speaking, the front portion 30-1 of the rear member 30 delimits at least a part of the mixing chamber 33 used for mixing the oxygen flow and the air flow(s), when required. More precisely, the rear member 30 comprises a hollow tubular portion 30-2 closed at one end by a blind bottom 30-3, i.e. it has a cup-like shape or similar as better shown in Fig. 7. The other end of the tubular portion 30-2 is open toward the inlet chamber 12 of the main body 10.

For instance, the rear member 30 has an outer diameter of about between 20 and 40 mm, and a length of about between 40 and 70 mm.

Further, the rear member 30 comprises, i.e. is traversed by, an oxygen feeding conduct 35 comprising an outer conduct portion 35-2, i.e. upstream portion, and an inner conduct portion 35-1, i.e. downstream portion, for providing oxygen gas to the venturi device 1.

The oxygen feeding conduct 35 is axially-arranged (XX axis) through the blind bottom 30-3 of the rear member 30, i.e. it traverses the blind bottom 30-3. The outer conduct portion 35-2 of the oxygen feeding conduct 35 comprises the oxygen inlet 31, at its distal free end, and is located outside of the venturi device 1.

Further, the inner conduct portion 35-1 of the oxygen feeding conduct 35 comprises an oxygen delivery port 36 for providing oxygen located at its free end.

The oxygen feeding conduct 35 is axially-traversed by a lumen 37 for conveying oxygen gas from the oxygen inlet 31 to the oxygen delivery port 36 thereby providing oxygen coming from an external oxygen source, such as a gas cylinder, a gas line or any other O₂ source.

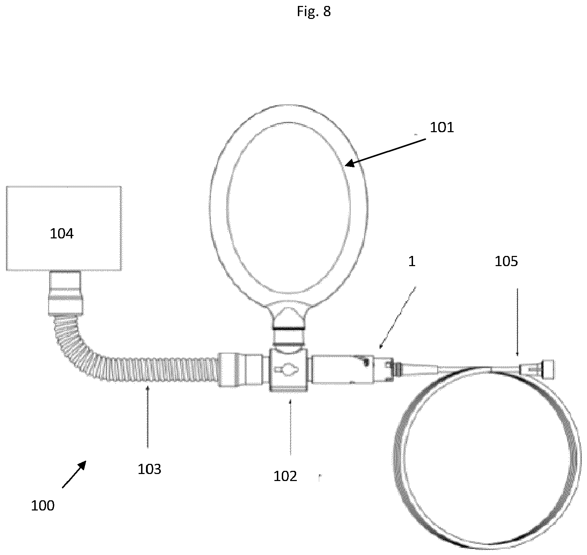

More precisely, the outer conduct portion 35-2 of the oxygen feeding conduct 35 also comprises connector means 38, such as a specific outer design, for fluidly connecting an oxygen line thereto as shown in Fig. 8, preferably an oxygen line fluidly connected to an external oxygen source, such as an oxygen gas cylinder or an oxygen wall-plug fed by a gas network.

As shown in Fig. 6A&6B and 7, the inner conduct portion 35-1 of the oxygen feeding conduct 35 is arranged so as to axially-project into the mixing chamber 33, whereas the outer conduct portion 35-2 of the oxygen feeding conduct 35 is arranged so as to axially-project outwardly of the rear member, i.e. outside of it.

The oxygen feeding conduct 35 is in fluid communication, in particular via the lumen 37 of the inner and outer conduct portions 35-1, 35-2, with the mixing chamber 33, when the rear member 30 is in the open position as shown in Fig. 6A and 7, for mixing air and oxygen, and providing such an O₂/air mixture to the venturi nozzle 20.

In contrast, the oxygen feeding conduct 35 is in fluid communication, in particular via the lumen 37, with the inner nozzle channel 20-1, 20-2 of the venturi nozzle 20, when the rear member 30 is in the closed position as shown in Fig. 6B. In the closed position, the proximal free end of the inner conduct portion 35-1 of the oxygen feeding conduct 35 penetrates in the inner nozzle channel 20-1, 20-2 of the venturi nozzle 20, through the nozzle inlet 21, to deliver oxygen directly into the venturi nozzle 20.

Figure 8 shows an installation 100 for providing oxygen or an air/oxygen mixture to a patient comprising a gas reservoir 101, a flexible hose 103, a respiratory interface 104, a connecting hollow body 102 and a venturi device 1 according to the present disclosure that are in fluid communication.

The venturi device 1 according to the present disclosure is fed with oxygen by an oxygen line 105, such as a flexible hose or the like, that conveys an oxygen flow provided by an oxygen source (not shown), such as an O₂ gas cylinder or an O₂ gas conduct.

As above explained, the venturi device 1 provides a gaseous flow of oxygen or of an air/O₂ mixture to the connecting hollow body 102 that itself feeds the gaseous flow to the gas reservoir 101, such as a flexible bag or the like, and/or to the flexible hose 103, such as a polymer hose, that conveys said gaseous flow to the respiratory interface 104, such as a respiratory mask, i.e. a nasal or facial mask, for delivering said oxygen or air/oxygen mixture to a patient in need thereof.

The gas reservoir constitutes a flexible buffer or the like having an inner volume of between about 1 and 50 L (L in water equivalent), for instance of about 30 L.

The installation 100 of the present disclosure can be used for providing oxygen or an air/oxygen mixture to a patient suffering from respiratory troubles, such as a person infected by a coronavirus, for example the Covid-19 virus or one of its variants, involving pulmonary troubles, failures or the like, leading to hypoxemia or the like.

## Claims

1. **Venturi** device (1) for delivering oxygen or an air/oxygen mixture comprising:
- a main body (10) comprising an inner compartment (11) and a venturi nozzle (20) arranged in said inner compartment (11),
- a rear member (30) comprising an oxygen inlet (31) for providing oxygen and at least one air inlet (32) for providing air, and a mixing chamber (33) for mixing therein oxygen and air provided by said oxygen inlet (31) and said at least one air inlet (32), said rear member (30) being mobile with respect to the main body (10) between at least :
o a closed position wherein the rear member (30) provides oxygen to the venturi nozzle (20), and
o an open position wherein the rear member (30) provides an air/oxygen mixture to the venturi nozzle (20), said air/oxygen mixture being obtained in the mixing chamber (33),
**characterized in that** the rear member (30) is mobile with respect to the main body (10) according to a helicoidal motion for allowing a user to manually select a desired open or closed position.

2. Venturi device according to Claim 1, **characterized in that** the main body (10) comprises at least one elongated groove (40) and the rear member (30) comprises at least one guiding member (50), or vice versa, said at least one guiding member (50) being inserted into said at least one elongated groove (40) so as to cooperate with said at least one elongated groove (40) for guiding the motion of the rear member (30) with respect to the main body (10), while the rear member (30) is actuated by a user.

3. Venturi device according to Claim 2, **characterized in that** the main body (10) comprises at least two elongated grooves (40) and the rear member (30) comprises at least two guiding members (50), or vice versa.

4. Venturi device according to Claim 1, **characterized in that** the inner compartment (11) of the main body (10) comprises an inlet chamber (12) and a gas outlet (13), the inlet chamber (12) of the main body (10) being in fluid communication with the mixing chamber (33) of the rear member (30), and a front portion (30-1) of the rear member (30) is inserted into at least a part (12-1) of the inlet chamber (12) of the main body (10).

5. Venturi device according to any one of Claims 2 or 3, **characterized in that** :
- the elongated grooves (40) are slots (41) arranged in the peripheral wall (42) of the main body (10), and
- the guiding members (50) are or comprise protrusions (51) arranged on the outer surface of the rear member (30).

6. Venturi device according to Claims 2, 3 or 5, **characterized in that** each elongated groove (40), in particular each slot (41), comprises a curved linear portion (47) terminated by two end lodgings (46A, 46B) each configured for lodging a guiding member (50) in the open or closed positions.

7. Venturi device according to Claim 6, **characterized in that** the curved linear portion (47) of each elongated groove (40) is diagonally-arranged in the peripheral wall (42) of the main body (10).

8. Venturi device according to Claim 6, **characterized in that** each end lodging (46A, 46B) comprises an abutment (45).

9. Venturi device according to Claim 1, **characterized in that** the rear member (30) comprises an inner conduct element (35) comprising a proximal portion (35-1) comprising an oxygen delivery port (36) and projecting into the mixing chamber (33) of the rear member (30).

10. Venturi device according to Claim 9, **characterized in that**, in the closed position, the proximal portion (35-1) of the inner conduct element (35) of the rear member (30) is directly fluidly connected to the inner nozzle channel (20-1, 20-2) of the venturi nozzle (20) for providing oxygen thereto.

11. Venturi device according to Claim 9, **characterized in that**, in the open position, the proximal portion (35-1) of the inner conduct element (35) of the rear member (30) opens into the mixing chamber (33) of the rear member (30) for providing oxygen therein and obtaining an air/oxygen mixture that is subsequently fed to the inner nozzle channel (20-1, 20-2) of the venturi nozzle (20).

12. Venturi device according to any one of the previous claims, **characterized in that** the guiding members (50) and the elongated grooves (40) are configured so that each guiding member (50) travels into one corresponding elongated groove (40), when the rear member (30) is actuated, clockwise or counterclockwise, with respect to the main body (10), or vice versa, by a user.

13. Venturi device according to claims 6 and 8, **characterized in that** the abutments (45) are configured for maintaining the guiding members (50) in the end lodgings (46A, 46B), in the open or closed positions.

14. Installation (100) for providing oxygen or an air/oxygen mixture to a patient comprising a gas reservoir (101), a flexible hose (103), a respiratory interface (104) and a connecting hollow body (102), the gas reservoir (101) and the flexible hose (103) being fluidly connected to the connecting hollow body (102), and the respiratory interface (104) being fluidly connected to the flexible hose (103), **characterized in that** a venturi device (1) according to any one of the preceding claims is further fluidly connected to the connecting hollow body (102) for delivering oxygen or the air/oxygen mixture to said connecting hollow body (102).

15. Installation (100) according to Claim 14, **characterized in that** an oxygen line (105) is fluidly connected to the venturi device (1) for providing oxygen thereto.

## Patentansprüche

1. Venturi-Vorrichtung (1) zum Zuführen von Sauerstoff oder einem Luft/Sauerstoff-Gemisch, umfassend:
- einen Hauptkörper (10), der eine innere Kammer (11) und eine in der inneren Kammer (11) angeordnete Venturi-Düse (20) umfasst,
- ein hinteres Glied (30), das einen Sauerstoffeinlass (31) zum Bereitstellen von Sauerstoff und mindestens einen Lufteinlass (32) zum Bereitstellen von Luft umfasst, sowie eine Mischkammer (33) zum Mischen von Sauerstoff und Luft darin, die durch den Sauerstoffeinlass (31) und den mindestens einen Lufteinlass (32) bereitgestellt worden sind, wobei das hintere Glied (30) bezüglich des Hauptkörpers (10) zwischen mindestens:
o einer geschlossenen Position, in der das hintere Glied (30) der Venturi-Düse (20) Sauerstoff zuführt, und
o einer offenen Position beweglich ist, in der das hintere Glied (30) der Venturi-Düse (20) ein Luft/Sauerstoff-Gemisch bereitstellt, wobei das Luft/Sauerstoff-Gemisch in der Mischkammer (33) erhalten wird,
**dadurch gekennzeichnet, dass** das hintere Glied (30) gemäß einer spiralförmigen Bewegung bezüglich des Hauptkörpers (10) beweglich ist, damit ein Benutzer eine gewünschte offene oder geschlossene Position manuell auswählen kann.

2. Venturi-Vorrichtung nach Anspruch 1, **dadurch gekennzeichnet, dass** der Hauptkörper (10) mindestens eine längliche Nut (40) umfasst und das hintere Glied (30) mindestens ein Führungsglied (50) umfasst, oder umgekehrt, wobei das mindestens eine Führungsglied (50) in die mindestens eine längliche Nut (40) eingeführt ist, um mit der mindestens einen länglichen Nut (40) zusammenzuwirken, um die Bewegung des hinteren Glieds (30) bezüglich des Hauptkörpers (10) zu führen, während das hintere Glied (30) von einem Benutzer betätigt wird.

3. Venturi-Vorrichtung nach Anspruch 2, **dadurch gekennzeichnet, dass** der Grundkörper (10) mindestens zwei längliche Nuten (40) umfasst und das hintere Glied (30) mindestens zwei Führungsglieder (50) umfasst, oder umgekehrt.

4. Venturi-Vorrichtung nach Anspruch 1, **dadurch gekennzeichnet, dass** die innere Kammer (11) des Hauptkörpers (10) eine Einlasskammer (12) und einen Gasauslass (13) umfasst, wobei die Einlasskammer (12) des Hauptkörpers (10) mit der Mischkammer (33) des hinteren Glieds (30) in Fluidverbindung steht und ein vorderer Abschnitt (30-1) des hinteren Glieds (30) in mindestens einen Teil (12-1) der Einlasskammer (12) des Hauptkörpers (10) eingeführt ist.

5. Venturi-Vorrichtung nach einem der Ansprüche 2 oder 3, **dadurch gekennzeichnet, dass**:
- die länglichen Nuten (40) Schlitze (41) sind, die in der Umfangswand (42) des Hauptkörpers (10) angeordnet sind, und
- die Führungsglieder (50) Vorsprünge (51) sind oder umfassen, die an der Außenfläche des hinteren Glieds (30) angeordnet sind.

6. Venturi-Vorrichtung nach Anspruch 2, 3 oder 5, **dadurch gekennzeichnet, dass** jede längliche Nut (40), insbesondere jeder Schlitz (41), einen gekrümmten linearen Abschnitt (47) umfasst, der durch zwei Endaufnahmen (46A, 46B) abgeschlossen ist, die jeweils zur Aufnahme eines Führungsglieds (50) in der offenen oder geschlossenen Position ausgestaltet sind.

7. Venturi-Vorrichtung nach Anspruch 6, **dadurch gekennzeichnet, dass** der gekrümmte lineare Abschnitt (47) jeder länglichen Nut (40) diagonal in der Umfangswand (42) des Hauptkörpers (10) angeordnet ist.

8. Venturi-Vorrichtung nach Anspruch 6, **dadurch gekennzeichnet, dass** jede Endaufnahme (46A, 46B) einen Anschlag (45) umfasst.

9. Venturi-Vorrichtung nach Anspruch 1, **dadurch gekennzeichnet, dass** das hintere Glied (30) ein inneres Leitelement (35) umfasst, das einen proximalen Abschnitt (35-1) umfasst, der eine Sauerstoffzufuhröffnung (36) umfasst und in die Mischkammer (33) des hinteren Glieds (30) ragt.

10. Venturi-Vorrichtung nach Anspruch 9, **dadurch gekennzeichnet, dass** der proximale Abschnitt (35-1) des inneren Leitelements (35) des hinteren Glieds (30) in der geschlossenen Position direkt mit dem inneren Düsenkanal (20-1, 20-2) der Venturi-Düse (20) fluidverbunden ist, um dieser Sauerstoff zuzuführen.

11. Venturi-Vorrichtung nach Anspruch 9, **dadurch gekennzeichnet, dass** der proximale Abschnitt (35-1) des inneren Leitelements (35) des hinteren Glieds (30) in der offenen Position in die Mischkammer (33) des hinteren Glieds (30) mündet, um dort Sauerstoff bereitzustellen und ein Luft/Sauerstoff-Gemisch zu erhalten, das anschließend dem inneren Düsenkanal (20-1, 20-2) der Venturi-Düse (20) zugeführt wird.

12. Venturi-Vorrichtung nach einem der vorhergehenden Ansprüche, **dadurch gekennzeichnet, dass** die Führungsglieder (50) und die länglichen Nuten (40) so ausgestaltet sind, dass jedes Führungsglied (50) in eine entsprechende längliche Nut (40) einfährt, wenn das hintere Glied (30) von einem Benutzer im Uhrzeigersinn oder entgegen dem Uhrzeigersinn bezüglich des Hauptkörpers (10) betätigt wird, oder umgekehrt.

13. Venturi-Vorrichtung nach Ansprüchen 6 und 8, **dadurch gekennzeichnet, dass** die Anschläge (45) dazu ausgestaltet sind, die Führungsglieder (50) in der offenen oder der geschlossenen Position in den Endaufnahmen (46A, 46B) zu halten.

14. Anlage (100) zum Bereitstellen von Sauerstoff oder einem Luft/Sauerstoff-Gemisch für einen Patienten, die ein Gasreservoir (101), einen flexiblen Schlauch (103), eine Atemschnittstelle (104) und einen Verbindungshohlkörper (102) umfasst, wobei das Gasreservoir (101) und der flexible Schlauch (103) mit dem Verbindungshohlkörper (102) fluidverbunden sind, und wobei die Atemschnittstelle (104) mit dem flexiblen Schlauch (103) fluidverbunden ist, **dadurch gekennzeichnet, dass** eine Venturi-Vorrichtung (1) nach einem der vorhergehenden Ansprüche ferner mit dem Verbindungshohlkörper (102) fluidverbunden ist, um dem Verbindungshohlkörper (102) Sauerstoff oder das Luft/Sauerstoff-Gemisch zuzuführen.

15. Anlage (100) nach Anspruch 14, **dadurch gekennzeichnet, dass** eine Sauerstoffleitung (105) mit der Venturi-Vorrichtung (1) fluidverbunden ist, um dieser Sauerstoff zuzuführen.

## Revendications

1. Dispositif venturi (1) destiné à délivrer de l'oxygène ou un mélange air/oxygène comprenant :
- un corps principal (10) comprenant un compartiment interne (11) et une buse venturi (20) disposée dans ledit compartiment interne (11),
- un élément arrière (30) comprenant une entrée d'oxygène (31) pour fournir de l'oxygène et au moins une entrée d'air (32) pour fournir de l'air, et une chambre de mélange (33) pour mélanger dans celle-ci l'oxygène et l'air fournis par ladite entrée d'oxygène (31) et ladite au moins une entrée d'air (32), ledit élément arrière (30) étant mobile par rapport au corps principal (10) entre au moins :
o une position fermée dans laquelle l'élément arrière (30) fournit de l'oxygène à la buse venturi (20), et
∘ une position ouverte dans laquelle l'élément arrière (30) fournit un mélange air/oxygène à la buse venturi (20), ledit mélange air/oxygène étant obtenu dans la chambre de mélange (33),
**caractérisé en ce que** l'élément arrière (30) est mobile par rapport au corps principal (10) selon un mouvement hélicoïdal pour permettre à un utilisateur de sélectionner manuellement une position ouverte ou fermée souhaitée.

2. Dispositif venturi selon la revendication 1, **caractérisé en ce que** le corps principal (10) comprend au moins une rainure allongée (40) et l'élément arrière (30) comprend au moins un élément de guidage (50), ou inversement, ledit au moins un élément de guidage (50) étant inséré dans ladite au moins une rainure allongée (40) de manière à coopérer avec ladite au moins une rainure allongée (40) pour guider le mouvement de l'élément arrière (30) par rapport au corps principal (10), pendant que l'élément arrière (30) est actionné par un utilisateur.

3. Dispositif venturi selon la revendication 2, **caractérisé en ce que** le corps principal (10) comprend au moins deux rainures allongées (40) et l'élément arrière (30) comprend au moins deux éléments de guidage (50), ou inversement.

4. Dispositif venturi selon la revendication 1, **caractérisé en ce que** le compartiment interne (11) du corps principal (10) comprend une chambre d'entrée (12) et une sortie de gaz (13), la chambre d'entrée (12) du corps principal (10) étant en communication fluidique avec la chambre de mélange (33) de l'élément arrière (30), et une partie avant (30-1) de l'élément arrière (30) est insérée dans au moins une partie (12-1) de la chambre d'entrée (12) du corps principal (10).

5. Dispositif venturi selon l'une quelconque des revendications 2 ou 3, **caractérisé en ce que** :
- les rainures allongées (40) sont des fentes (41) disposées dans la paroi périphérique (42) du corps principal (10), et
- les éléments de guidage (50) sont ou comprennent des protubérances (51) disposées sur la surface externe de l'élément arrière (30).

6. Dispositif venturi selon les revendications 2, 3 ou 5, **caractérisé en ce que** chaque rainure allongée (40), en particulier chaque fente (41), comprend une partie curviligne (47) terminée par deux logements d'extrémité (46A, 46B) configurés chacun pour loger un élément de guidage (50) dans les positions ouverte ou fermée.

7. Dispositif venturi selon la revendication 6, **caractérisé en ce que** la partie curviligne (47) de chaque rainure allongée (40) est disposée en diagonale dans la paroi périphérique (42) du corps principal (10).

8. Dispositif venturi selon la revendication 6, **caractérisé en ce que** chaque logement d'extrémité (46A, 46B) comprend une butée (45).

9. Dispositif venturi selon la revendication 1, **caractérisé en ce que** l'élément arrière (30) comprend un élément de conduite interne (35) comprenant une partie proximale (35-1) comprenant un orifice de distribution d'oxygène (36) et faisant saillie dans la chambre de mélange (33) de l'élément arrière (30).

10. Dispositif venturi selon la revendication 9, **caractérisé en ce que**, en position fermée, la partie proximale (35-1) de l'élément de conduite interne (35) de l'élément arrière (30) est en liaison fluidique directe avec le canal de buse interne (20-1, 20-2) de la buse venturi (20) pour fournir de l'oxygène à celle-ci.

11. Dispositif venturi selon la revendication 9, **caractérisé en ce que**, en position ouverte, la partie proximale (35-1) de l'élément de conduite interne (35) de l'élément arrière (30) débouche dans la chambre de mélange (33) de l'élément arrière (30) pour apporter de l'oxygène dans celle-ci et obtenir un mélange air/oxygène qui est ensuite acheminé vers le canal de buse interne (20-1, 20-2) de la buse venturi (20).

12. Dispositif venturi selon l'une quelconque des revendications précédentes, **caractérisé en ce que** les éléments de guidage (50) et les rainures allongées (40) sont configurés de sorte que chaque organe de guidage (50) se déplace dans une rainure allongée (40) correspondante, lorsque l'élément arrière (30) est actionné, dans le sens des aiguilles d'une montre ou dans le sens inverse, par rapport au corps principal (10), ou inversement, par un utilisateur.

13. Dispositif venturi selon les revendications 6 et 8, **caractérisé en ce que** les butées (45) sont configurées pour maintenir les éléments de guidage (50) dans les logements d'extrémité (46A, 46B), en position ouverte ou fermée.

14. Installation (100) destinée à fournir de l'oxygène ou un mélange air/oxygène à un patient comprenant un réservoir de gaz (101), un tuyau flexible (103), une interface respiratoire (104) et un corps creux de raccordement (102), le réservoir de gaz (101) et le tuyau flexible (103) étant raccordés fluidiquement au corps creux de raccordement (102), et l'interface respiratoire (104) étant raccordée fluidiquement au tuyau flexible (103), **caractérisée en ce qu'**un dispositif venturi (1) selon l'une quelconque des revendications précédentes est en outre raccordé fluidiquement au corps creux de raccordement (102) pour délivrer de l'oxygène ou le mélange air/oxygène audit corps creux de raccordement (102).

15. Installation (100) selon la revendication 14, **caractérisée en ce qu'**une ligne d'oxygène (105) est raccordée fluidiquement au dispositif venturi (1) pour fournir de l'oxygène à celui-ci.
